# EUROPEAN PATENT APPLICATION

(11) **EP 4 803 980 A1**
(43) Date of publication of application: **09.09.2026**
(21) Application number: 26151268.5
(22) Date of filing: 12.01.2026
(51) Int. Cl.: G06F 3/01, A61B 5/18, B64D 43/00, G01C 23/00, G06Q 10/0631

(54) **SYSTEMS AND METHODS FOR COGNITIVE STATE ESTIMATION**

(30) Priority: 03.03.2025 US 202519068195
(71) Applicant: The Boeing Company, Arlington, VA 22202 (US)
(72) Inventor: BURKS, Charles Luke, Arlington 22202 (US); GREENE, Max L., Arlington 22202 (US)
(74) Representative: Boult Wade Tennant LLP

(57) **Abstract**

A system for automatically assigning tasks includes a plurality of sensors configured to monitor an operator and generate physiological data associated with the operator. The system also includes one or more processors configured to determine a cognitive state estimate for the operator based on the physiological data associated with the operator, identify a task list associated with a set of tasks assigned to the operator, and identify one or more user metrics associated with the operator. The one or more processors are also configured to, based on the cognitive state estimate, the task list, and the one or more user metrics, assign a task from a set of assignable tasks to the operator. The one or more processors are also configured to and send, to an interface device associated with the operator, a task assignment indicator that indicates the task is assigned to the operator.

## Description

### FIELD OF THE DISCLOSURE

The present disclosure is generally related to systems and methods for cognitive state estimation.

### BACKGROUND

Operators, such as operators of an aircraft, can experience various cognitive challenges while working, including mental fatigue, which can lead to critical errors in task completion. In operating environments in which safety is paramount, such as flight operation tasks for an aircraft, those errors can lead to critical errors in flight operations, reducing safety. Cognitive discrepancies among operators can stem from a variety of factors, including mental fatigue, high workload, attentional lapses, and the limitations of human information processing in dynamic, multitasking environments.

Certain previous approaches to addressing these issues relied on qualitative estimates of an operator's cognitive state by a human supervisor and/or standardized training and procedural measures, without incorporating real-time physiological data or personalized cognitive assessments. These types of methods have not considered the unique cognitive and physiological characteristics of each operator. This lack of personalized data has limited the ability to accurately detect and respond to cognitive challenges as they arise during operations.

The potential for such cognitive misalignments poses significant risks to organizational efficiency and efficacy, particularly in situations such as flight operations that require rapid decision-making. Without a way to account for individual differences between operators, it has been challenging to develop effective real-time interventions that can mitigate these risks.

### SUMMARY

There is described herein a system for automatically assigning tasks includes a first plurality of sensors and one or more processors. The first plurality of sensors is configured to monitor a first operator and generate first physiological data associated with the first operator. The one or more processors are configured to determine a first cognitive state estimate for the first operator based on the first physiological data associated with the first operator, identify a first task list associated with a first set of tasks assigned to the first operator, and identify one or more first user metrics associated with the first operator. The one or more processors are also configured to, based on the first cognitive state estimate, the first task list, and the one or more first user metrics, assign a first task from a set of assignable tasks to the first operator. The one or more processors are further configured to and send, to an interface device associated with the first operator, a first task assignment indicator that indicates the first task is assigned to the first operator.

There is described herein a method includes receiving, from a first plurality of sensors configured to monitor a first operator, first physiological data generated by the first plurality of sensors. The first physiological data is associated with the first operator. The method also includes determining a first cognitive state estimate for the first operator based on the first physiological data associated with the first operator. The method further includes identifying a first task list associated with a first set of tasks assigned to the first operator. The method includes identifying one or more first user metrics associated with the first operator. The method also includes, based on the first cognitive state estimate, the first task list, the one or more first user metrics, or a combination thereof, assigning a first task from a set of assignable tasks to the first operator. The method further includes sending, to an interface device associated with the first operator, a first task assignment indicator that indicates the first task is assigned to the first operator.

There is described herein a non-transitory computer-readable medium storing instructions that, when executed by one or more processors, cause the one or more processors to receive, from a first plurality of sensors configured to monitor a first operator, first physiological data generated by the first plurality of sensors. The first physiological data is associated with the first operator. The instructions, when executed by the one or more processors, also cause the processors to determine a first cognitive state estimate for the first operator based on the first physiological data associated with the first operator. The instructions, when executed by the one or more processors, further cause the processors to identify a first task list associated with a first set of tasks assigned to the first operator. The instructions, when executed by the one or more processors, also cause the processors to identify one or more first user metrics associated with the first operator. The instructions, when executed by the one or more processors, cause the processors to, based on the first cognitive state estimate, the first task list, the one or more first user metrics, or a combination thereof, assign a first task from a set of assignable tasks to the first operator. The instructions, when executed by the one or more processors, further cause the processors to send, to an interface device associated with the first operator, a first task assignment indicator that indicates the first task is assigned to the first operator.

The features, functions, and advantages described herein can be achieved independently in various examples or may be combined in yet other examples, further details of which can be found with reference to the following description and drawings.

### BRIEF DESCRIPTION OF THE DRAWINGS

FIG. 1 is a diagram that illustrates an example automatic task assignment system.
FIG. 2 illustrates an example of the algorithm(s) estimator that includes a Multi-Modal Cognitive State Estimation Framework.
FIG. 3 is a diagram that illustrates a particular example of the algorithm(s) estimator that includes a Gaussian Mixture Model (GMM) algorithm.
FIG. 4 is a flow chart of a method of use of an automatic task assignment system.
FIG. 5 is a flowchart illustrating an example of a life cycle of an aircraft that includes the automatic task assignment system of FIG. 1.
FIG. 6 is a block diagram illustrating features of an illustrative aircraft that includes an automatic task assignment system.
FIG. 7 is a diagram of electronic components of an automatic task assignment system.

### DETAILED DESCRIPTION

Examples disclosed herein present systems, apparatus, and methods that monitor one or more operators, users, or other human workers to help improve the efficacy and efficiency of automated task assignments for those operators. The system uses various sensors to measure metrics, such as an operator's eye movements, heart rate, or skin responses, as illustrative, non-limiting examples. The system also collects data associated with one or more tasks, such as currently assigned tasks, tasks to be assigned, certain cost metrics associated with the set of assignable tasks, etc., that can automatically guide the system in the task assignment.

The physiological data associated with each of the operators is processed by the monitoring system that analyzes the data to determine a cognitive state estimate for each operator. The system looks at factors such as how tired or stressed the operator might be, how much mental workload the operator is experiencing, and how well the operator is paying attention to current task information. The system uses this information to detect, for example, whether the operator is likely experiencing mental fatigue. If the system detects that an operator might be experiencing mental fatigue when a new task is to be assigned, the system can assign the operator a new task that requires less mental focus than another task. The system can determine the task to be assigned based on the cognitive state estimate, as well as one or more user metrics such as an operator's skill level, task completion speed, cognitive state range, workload range, etc.

The system uses one or more algorithms to determine the cognitive state estimate of each operator, which is used to detect mental fatigue. For example, one or more of the algorithms can be configured to track what information the operator has likely seen based on where the operator has been looking. Another algorithm can estimate mental fatigue by comparing an operator's current user metric(s) (e.g., task completion speed) with historical data associated with those user metric(s) (e.g., historical task completion speed). These algorithms work together to build a comprehensive picture of the operator's awareness and the current situation.

By using the techniques and systems described herein, automated task assignment is improved as the automatic task assignment systems apply measured physiological data to automatically determine an operator's mental state rather than relying on more inaccurate and/or inconsistent monitoring methods such as observation by a human supervisor. The systems and methods disclosed herein can therefore improve the efficiency and efficacy of many different types of environments in which human operators are automatically assigned tasks.

The figures and the following description illustrate specific examples. It will be appreciated that those skilled in the art will be able to devise various arrangements that, although not explicitly described or shown herein, embody the principles described herein and are included within the scope of the claims that follow this description. Furthermore, any examples described herein are intended to aid in understanding the principles of the disclosure and are to be construed as being without limitation. As a result, this disclosure is not limited to the specific examples described below, but by the claims and their equivalents.

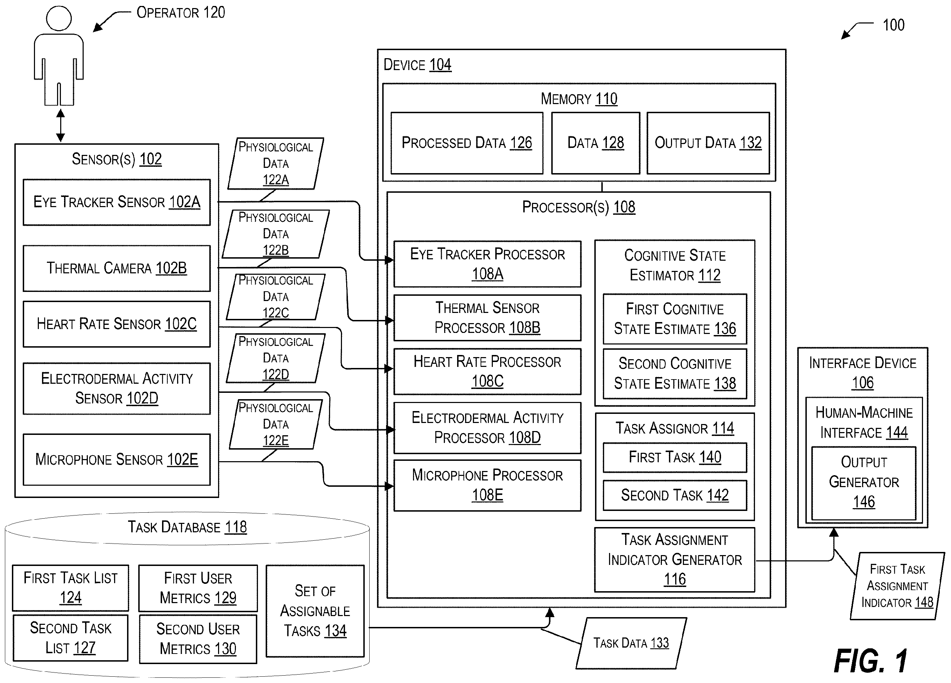

Particular examples are described herein with reference to the drawings. In the description, common features are designated by common reference numbers throughout the drawings. In some drawings, multiple instances of a particular type of feature are used. Although these features are physically and/or logically distinct, the same reference number is used for each, and the different instances are distinguished by addition of a letter to the reference number. When the features as a group or a type are referred to herein (e.g., when no particular one of the features is being referenced), the reference number is used without a distinguishing letter. However, when one particular feature of multiple features of the same type is referred to herein, the reference number is used with the distinguishing letter. For example, referring to FIG. 1, multiple sensors 102 are illustrated and associated with reference numbers 102A, 102B, 102C, 102D and 102E. When referring to a particular one of these sensors, such as the eye tracker sensor 102A, the distinguishing letter "A" is used. However, when referring to any arbitrary one of these sensors, the reference number 102 is used without a distinguishing letter.

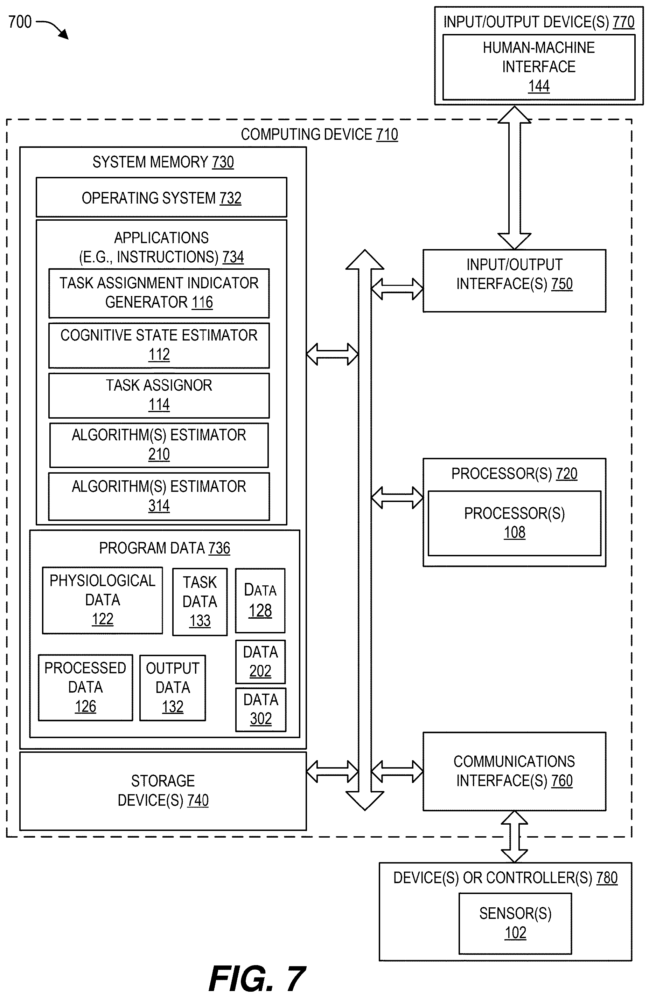

As used herein, various terminology is used for the purpose of describing particular examples only and is not intended to be limiting. For example, the singular forms "a," "an," and "the" are intended to include the plural forms as well, unless the context clearly indicates otherwise. Further, some features described herein are singular in some examples and plural in other examples. To illustrate, FIG. 7 depicts a computing device 710 including one or more processors ("processor(s)" 720 in FIG. 9), which indicates that in some examples the computing device 710 includes a single processor 720 and in other examples the computing device 710 includes multiple processors 720. For ease of reference herein, such features are generally introduced as "one or more" features and are subsequently referred to in the singular or optional plural (as typically indicated by "(s)") unless features related to multiple of the features are being described.

The terms "comprise," "comprises," and "comprising" are used interchangeably with "include," "includes," or "including." Additionally, the term "wherein" is used interchangeably with the term "where." As used herein, "exemplary" indicates an example, and should not be construed as limiting or as indicating a preference or a preferred example. As used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). As used herein, the term "set" refers to a grouping of one or more elements, and the term "plurality" refers to multiple elements.

As used herein, "generating," "calculating," "using," "selecting," "accessing," and "determining" are interchangeable unless context indicates otherwise. For example, "generating," "calculating," or "determining" a parameter (or a signal) can refer to actively generating, calculating, or determining the parameter (or the signal) or can refer to using, selecting, or accessing the parameter (or signal) that is already generated, such as by another component or device. As used herein, "coupled" can include "communicatively coupled," "electrically coupled," or "physically coupled," and can also (or alternatively) include any combinations thereof. Two devices (or components) can be coupled (e.g., communicatively coupled, electrically coupled, or physically coupled) directly or indirectly via one or more other devices, components, wires, buses, networks (e.g., a wired network, a wireless network, or a combination thereof), etc. Two devices (or components) that are electrically coupled can be included in the same device or in different devices and can be connected via electronics, one or more connectors, or inductive coupling, as illustrative, non-limiting examples. **In** some examples, two devices (or components) that are communicatively coupled, such as in electrical communication, can send and receive electrical signals (digital signals or analog signals) directly or indirectly, such as via one or more wires, buses, networks, etc. As used herein, "directly coupled" is used to describe two devices that are coupled (e.g., communicatively coupled, electrically coupled, or physically coupled) without intervening components.

FIG. 1 is a diagram that illustrates an example automatic task assignment system 100. The system 100 includes one or more sensors 102, a task database 118, a device 104 coupled to the sensor(s) 102, and an interface device 106. The device 104 includes processor(s) 108 coupled to a memory 110. The processor(s) 108 include a cognitive state estimator 112, a task assignor 114, and a task assignment indicator generator 116. The system 100 can be included in a computing device or in a distributed computing system with components integrated with each other or remote for one another. The components of the system 100 can be a part of, integrated into, or otherwise associated with a stationary computing device, mobile computing device, a vehicle (e.g., aircraft, a landcraft, a watercraft, a spacecraft, etc.), or other appropriate computing environment.

The system 100 can be configured to use procedures to enhance an accuracy and reliability of the system 100 in determining a cognitive state estimate for one or more operators 120 of the system 100. For example, the system 100 can be configured to use physiological baselining and calibration for environmental factors.

Physiological baselining can be utilized to reduce the impact of confounding variables and can account for physiological differences across operators 120. This approach can help mitigate the effects of nervousness or stress induced by participating in a data collection event, by allowing the operator 120 a short duration of time to become acclimated to the test environment. Physiological data can be taken for a short duration while the operator 120 is in a state of rest; this can allow the system 100 to derive additional variables during data collection, representing the difference in the physiological data from baseline. For example, as will be explained in more detail below, the system 100 can be configured to leverage variables such as "heart_rate_difference", which represents the difference between the instantaneous heart rate and the average heart rate during baseline data collection. Using physiological baselining can help minimize inter- and intra-operator variation in the algorithms when predicting mental fatigue and cognitive workload by considering how the operator's 120 current physiology has deviated from their physiology at rest.

In addition to physiological baselining, the system 100 can be configured to employ calibration procedures to account for environmental factors, particularly the influence of lighting conditions on pupil diameter. Individual differences, such as age, can impact the pupillary response to variations in environmental lighting. To account for this, the system 100 can be configured to measure a subject's pupil diameter while they observe a screen with changing brightness levels. The resulting pairwise data, associating each brightness value with its corresponding pupil diameter measurement, can be used to fit a pupillary response curve specific to that individual operator 120. As will be described in more detail below with regards to processing data related to eye tracking data (e.g., physiological data 122A), this curve can be used during task assignment to estimate the influence of dynamic ambient lighting conditions on that operator's 120 pupil diameter. This can enable the system 100 to differentiate between pupil size variations due to cognitive factors, such as mental workload, and those due to environmental lighting conditions, thereby increasing the robustness of the cognitive state estimator 112.

The sensors 102 can include an eye tracker sensor 102A, a thermal camera 102B, a heart rate sensor 102C, an electrodermal activity sensor 102D, a microphone sensor 102E, or a combination thereof, as illustrative, non-limiting examples. While five sensors are depicted in FIG. 1, in other examples a different number (e.g., two, three, four, or some other number) of sensors 102 can be used. Additionally, although particular sensors are illustrated in FIG. 1, different sensors can be incorporated into the system 100 without departing from the scope of the subject disclosure. For example, the sensor(s) 102 can include an ambient light sensor, timer, a camera or video recorder, etc.

The eye tracker sensor 102A can be configured to monitor various aspects of the operator's 120 visual behavior. The eye tracker sensor 102A can be configured to measure gaze direction in three-dimensional space, enabling the device 104 to determine where the operator 120 is looking at any given moment. This enables the device 104 to determine which objects or displays the operator 120 has observed. The eye tracker sensor 102A can also be configured to measure pupil diameter (e.g., pupil dilation), which can be an indicator of cognitive workload or emotional state. In some examples, the eye tracker sensor 102A can be configured to track head position and orientation, providing information about the operator's 120 posture and general attention direction. The eye tracker sensor 102A can be configured to detect and analyze saccades (rapid eye movements), fixations (periods when the eyes are relatively still), and blinks, all of which can provide insights into the operator's 120 attention patterns and fatigue levels.

The eye tracker sensor 102A can be configured to send physiological data 122A (e.g., eye tracking data) to a processor 108 of the device 104. For example, the eye tracker sensor 102A can be configured to send the physiological data 122A to an eye tracker processor 108A to analyze the operator's 120 visual attention patterns and determine various cognitive states. In some examples, the eye tracker processor 108A can be configured to calculate metrics such as fixation duration, saccade frequency, and scan patterns across the cockpit instruments. These metrics help determine where the operator 120 is focusing their attention and how efficiently they are gathering visual information. For example, longer fixation durations on a particular instrument can indicate increased cognitive processing of that information, while frequent saccades between instruments can suggest high situational awareness or potentially information overload.

The sensor(s) 102 can also include one or more thermal cameras 102B. The one or more thermal camera(s) 102B can be configured to measure various heat-related factors associated with the operator 120 and/or the operator's environment. For example, the thermal camera(s) 102B can measure skin temperature, breathing rate, breathing depth, blood pressure, etc. This information enables the device 104 to determine the current physiological health of the operator 120, allowing for more accurate interpretation of the physiological data 122A by distinguishing between pupil changes caused by cognitive factors and those caused by other health-related factors. The one or more thermal cameras 102B can be configured to send physiological data 122B (e.g., thermal data) to the processor 108. For example, the one or more thermal cameras 102B can be configured to send the physiological data 122B to a thermal sensor processor 108B.

The heart rate sensor 102C can be configured to monitor the operator's 120 heart rate and heart rate variability. The heart rate sensor 102C can be configured to monitor the operator 120 continuously, at periodic intervals, or a combination thereof. These physiological signals can provide insights into the operator's 120 stress levels, workload, and overall physiological state. For example, changes in heart rate patterns can indicate increased cognitive load or the onset of fatigue, both of which can be factors in maintaining a safe working environment, safe flight operations, or a combination thereof.

In some examples, the heart rate sensor 102C can be a wearable device such as a wristband, ring, watch, and so forth. The heart rate sensor 102C can be configured to send physiological data 122C (e.g., heart rate data) to the processor 108. For example, the heart rate sensor 102C can be configured to send the physiological data 122C to a heart rate processor 108C, which processes the physiological data 122C to assess the operator's 120 stress levels and overall physiological arousal. In some examples, the heart rate processor 108C analyzes heart rate variability (HRV) metrics, such as the standard deviation of normal-to- normal (NN) intervals (SDNN) and the root mean square of successive R wave interval differences (RMSSD). Lower HRV might indicate increased stress or mental workload, while changes in HRV patterns over time can signal the onset of fatigue. For example, a sustained decrease in SDNN during a complex flight maneuver could suggest elevated cognitive load, while a gradual reduction in RMSSD over a long flight might indicate increasing fatigue.

The electrodermal activity (EDA) sensor 102D can be configured to measure changes in the electrical properties of the operator's 120 skin. Specifically, the EDA sensor 102D can be configured to track skin conductance, which tends to increase during periods of stress or heightened cognitive activity.

The device 104 uses the physiological data 122D (e.g., EDA data) to measure the operator's 120 physiological arousal and stress levels. In processing this physiological data 122D, the device 104 can be configured to identify skin conductance responses (SCRs) and analyze their frequency and amplitude. Increased SCR activity can indicate heightened stress or cognitive load, particularly when correlated with specific events or tasks. For example, a sudden increase in SCR frequency and amplitude (e.g., a rate of change that is greater than or equal to a threshold) during a particular task could indicate elevated stress levels, while sustained high SCR activity during a complex task might suggest high cognitive load.

The EDA sensor 102D can be configured to send the physiological data 122D (e.g., EDA data) to the sensor processor 108. For example, the EDA sensor 102D can be configured to send the physiological data 122D to an EDA processor 108D, which performs analyses of SCRs to contribute to the overall assessment of the operator's 120 physiological arousal and stress levels, as described in more detail herein.

The microphone sensor 102E can be configured to capture physiological data 122E (e.g., audio data) such as the operator's 120 speech, breathing, or a combination thereof. For example, the microphone sensor 102E can be configured to capture various aspects of vocal patterns, including frequency, communication intervals, and response times. As another example, the microphone sensor 102E can be configured to capture various aspects of breathing patterns, including frequency.

The device 104 can be configured to process the physiological data 122E to analyze the operator's 120 speech patterns in detail. The device 104 can be configured to examine features such as speech rate, pitch variation, and vocal tension. Changes in these parameters (e.g., metrics) can indicate increased stress or cognitive load. For example, a higher pitch and faster speech rate might suggest elevated stress levels, while longer response times or increased pauses might indicate higher cognitive load or fatigue.

The microphone sensor 102E can be configured to send the physiological data 122E (e.g., audio data) to the sensor processor 108. For example, the microphone sensor 102E can be configured to send the physiological data 122E to a microphone processor 108E, which performs these detailed analyses of speech patterns to contribute to the overall assessment of the operator's 120 cognitive state and stress levels, as described in more detail herein.

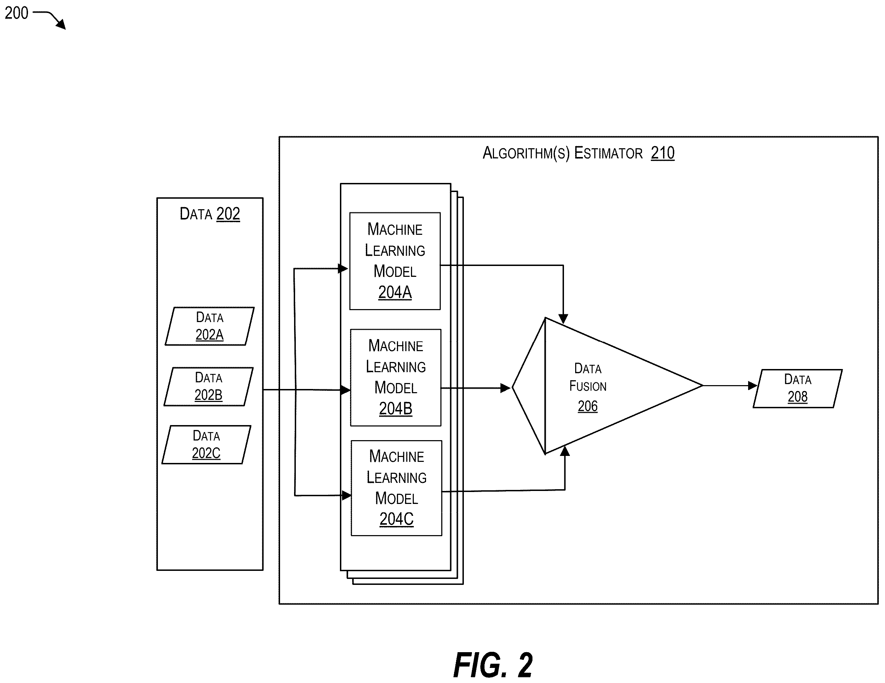

The device 104, in some examples, includes interfaces for each of the sensors 102 that preprocess the physiological data 122 before sending it to a respective processor 108. These interfaces and the initial preprocessing will be discussed in further detail in FIG. 2.

The eye tracker processor 108A can be configured to analyze the physiological data 122A from the eye tracker sensor 102A. The eye tracker processor 108A can be configured to determine fixation durations and frequencies, detect saccade patterns, and measure pupil diameter changes (e.g., a change in pupil dilation). The eye tracker processor 108A can be configured to calculate head position and orientation. In some examples, the eye tracker processor 108A can be configured to compute metrics such as PERCLOS (percentage of eyelid closure) for fatigue detection and gaze entropy for assessing situational awareness.

The thermal sensor processor 108B can be configured to normalize and calibrate the heat measurements from the physiological data 122B, converting sensor readings into standardized units of heat measurement. The thermal sensor processor 108B can be configured to detect changes in heat conditions that can indicate changes to an operator's physical environment that can affect an operator's thermal response to reduce noise in the physiological data 122B.

The heart rate processor 108C can be configured to analyze the physiological data 122C that includes cardiac signals from the heart rate sensor 102C. The heart rate processor 108C can be configured to calculate heart rate and heart rate variability (HRV). The heart rate processor 108C can be configured to compute metrics such as the standard deviation of NN intervals (SDNN) and the root mean square of successive R wave interval differences (RMSSD), which provide insights into the operator's 120 stress levels and autonomic nervous system activity.

The electrodermal activity (EDA) processor 108D can be configured to identify significant skin conductance responses (SCRs) from the physiological data 122D. The EDA processor 108D can be configured to calculate an amplitude and frequency of these responses and derive overall measures of sympathetic nervous system arousal. The EDA processor 108D can be configured to separate the tonic (baseline) and phasic (rapid-changing) components of the physiological data 122D, providing a nuanced view of the operator's 120 physiological arousal state.

The microphone processor 108E can be configured to analyze various aspects of the physiological data 122E from the microphone sensor 102E. The microphone processor 108E can be configured to measure fundamental frequency (pitch), analyze spectral characteristics, and potentially apply speech recognition algorithms to the physiological data 122E. The microphone processor 108E can be configured to calculate metrics like jitter and shimmer in the voice, which can be indicators of stress or fatigue. The microphone processor 108E can be configured to perform cepstral analysis to derive features like the cepstral peak prominence, which can be used to determine vocal fatigue.

After the physiological data 122 has been processed by one or more of the individual processors 108, the resulting processed data 126 is then sent to one or more cognitive state estimator 112 and the memory 110 (e.g., via a ZeroMQ (ZMQ) handler). This ensures that the cognitive state estimator 112 has access to the most current processed data 126 for real-time analysis, while also preserving the processed data 126 for later review, analysis, or potential reprocessing with improved algorithms. Although described as the physiological data 122 being processed by individual, respective processors 108, in other example, at least one processor 108 may process physiological data 122 received from multiple sensors, such as two or more different types of sensors.

The task database 118 can be configured to store various data that represents the current status of tasks assigned to various operators 120, current performance of the operator(s), future tasks to be assigned, etc. This includes, but is not limited to, a first task list 124 (e.g., a list of tasks currently assigned to a first operator), a second task list 127 (e.g., a list of tasks currently assigned to a second operator), first user metrics 129 (e.g., one or more user metrics associated with the current and/or past performance of the first operator), second user metrics 130 (e.g., one or more user metrics associated with the current and/or past performance of the second operator), and a set of assignable tasks 134 (e.g., a list of assignable tasks). The task database 118 can be configured to provide some or all of this data to the device 104 as task data 133.

In some examples, the task database 118 can be configured to interface with various systems and sensors to collect task data. For example, the task database 118 can be configured to gather information from a centralized depository of assignable tasks, operator task reporting software, etc.

The first task list 124 can include data associated with one or more tasks currently assigned to a first operator of the operators 120. The second task list 127 can include data associated with a second operator of the operators 120. For example, the task lists 124, 127 can include data indicative of a working task, a waiting task, or a combination thereof.

The user metrics 129, 130 can include data associated with one or more user metrics associated with a particular operator. For example, the user metrics 129, 130 can include data indicative of an operator's skill level, task completion speed, cognitive state range, workload range, or a combination thereof.

The cognitive state estimator 112 can be configured to perform several functions once it receives the processed data 126. The cognitive state estimator 112 can be configured to integrate the diverse data streams to build a comprehensive picture of the operator's 120 cognitive state. For example, if the physiological data 122A shows rapid scanning between instruments, the physiological data 122C indicates elevated stress levels, and the physiological data 122E suggests increased tension, the cognitive state estimator 112 can determine that the operator 120 is experiencing high mental workload and potentially approaching cognitive overload.

In some examples, the cognitive state estimator 112 includes multiple Kalman filters and a Gaussian Mixture Model (GMM) algorithm. Each Kalman filter represents a different hypothesis about a cognitive state of the operator 120, accounting for individual variations in operator responses. The GMM algorithm can be configured to combine the outputs from these multiple Kalman filters, allowing for a probabilistic representation of the cognitive state of the operator 120 that captures both the most likely state and the uncertainty in the estimate.

The GMM algorithm can be configured to combine weighted outputs of the multiple Kalman filters. Each filter's output can be represented as a Gaussian component within a mixture, and the weights assigned to these components can be dynamically adjusted based on the physiological data 122 and the task data 133. This dynamic weighting mechanism can enable the device 104 to adapt its estimates to the individual characteristics of the operator 120 being monitored and to dynamic task data.

In some examples, the physiological data 122 and/or the task data 133 can be used to update both the individual Kalman filters and their respective weights in the GMM algorithm. This adaptive approach can enable the device 104 to provide a more accurate and nuanced estimation of the cognitive state of the operator 120 over time, enhancing an ability of the device 104 to detect potential cognitive strain or other cognitive issues that could affect performance of the operator 120.

The cognitive state estimator 112 can be configured to include a compound data fusion scheme. The compound data fusion scheme enables the cognitive state estimator 112 to include multiple interdependent estimation algorithms within the context of a Probabilistic Graphical Model (PGM) algorithm. This approach enables the cognitive state estimator 112 to leverage the strengths of different analytical techniques while maintaining a coherent probabilistic framework.

Within the Multi-Modal Cognitive State Estimation Framework, various machine learning models can be employed to process different aspects of the processed data 126. For example, a neural network can be used to classify eye movement patterns, while a Bayesian inference model algorithm could estimate fatigue levels based on the processed data 126 (e.g., physiological data). The outputs from each algorithm can be weighted according to their explanatory power for the given human state and fused into a single probabilistic estimate. The outputs of these individual models can then integrate within the PGM algorithm, which represents the relationships between different cognitive states and observable data as a graph structure.

The cognitive state estimator 112 can be configured to include a probabilistic perception estimation algorithm. This approach leverages the sequential nature of gaze data (e.g., processed data 126A) to create probabilistic perception estimates of discrete gaze events with quantified uncertainty. **It** can use weighted aggregation of raw gaze measurements over time windows, subdivided by saccades, to remove epistemic uncertainty and produce a 3D probabilistic view cone. This cone can then project onto a two-dimensional (2D) surface along with world model objects to calculate probabilistic object intersections.

The cognitive state estimator 112 can be configured to include a Multiple Model Cognitive State Estimator algorithm. The Multiple Model Cognitive State Estimator algorithm can provide cognitive state estimates for the operator 120 being monitored. The algorithm can begin with a set of pre-trained cognitive estimation models, each learned from historical data of various operators 120 using an Expectation Maximization model approach. These models can represent different patterns of how physiological signals relate to cognitive states. Each pre-trained model can be implemented as the measurement likelihood function in a separate Kalman filter, using a Nearly Constant Position dynamics model that assumes cognitive states change slowly over time unless perturbed by new observations.

In some examples, as the Multiple Model Cognitive State Estimator algorithm receives new data (e.g., processed data 126) for the current operator 120, the device 104 (e.g., the processor 108) can run multiple Kalman filters in parallel. The outputs of these parallel filters can then be combined using a dynamic weighting scheme, where weights can be calculated based on how well each model's predictions match the incoming data (e.g., processed data 126) from a particular operator. This enables the cognitive state estimator 112 to adapt its estimates to the individual characteristics of the operator 120 being monitored. The collection of weighted filter outputs is represented as a GMM algorithm, providing a probabilistic estimate of the operator's 120 cognitive state that captures both the most likely state and the uncertainty in the estimate.

As more data (e.g., processed data 126) is collected from the operator 120 (e.g., as the operator 120 completes additional tasks), the weighting of different models can be continuously updated. This ongoing refinement enables the cognitive state estimator 112 to adapt its estimates over time, tailoring them to the specific patterns exhibited by the current operator.

The cognitive state estimator 112 can employ a combination of these algorithms in a modular and flexible manner to process the physiological data 122 and the task data 133. Depending on a particular implementation and/or requirement(s), the cognitive state estimator 112 can include and use all of these algorithms in concert or select a subset of them. For example, the GMM algorithm can be configured to combine outputs from multiple Kalman filters, while a pre-corrected Fast Fourier Transform (pFFT) algorithm can be configured to provide context about the task data that can inform another algorithm's interpretations. A probabilistic graphical model (PGM) can integrate outputs from various other algorithms to build a comprehensive model of the operator's awareness. This modular approach can enable the system 100 to be adaptable to different scenarios and requirements.

After processing the processed data 126 using this flexible combination of algorithms, the cognitive state estimator 112 can be configured to communicate a cognitive state estimate (e.g., a first cognitive state estimate 136 associated with the first operator and a second cognitive state estimate 138 associated with the second operator) to the task assignor 114. The cognitive state estimate can include an estimate of a variety of operator cognitive state functions such as a fatigue of the operator, a task engagement level, etc., or a combination thereof.

The task assignor 114 can be configured to, based on the cognitive state estimate and the task data 133, assign a task to the operator(s) 120. For example, the task assignor 114 can be configured to, based on the first cognitive state estimate 136, the first task list 124, and the one or more first user metrics 129, assign a first task 140 from the set of assignable tasks 134 to the first operator. The task assignor 114 can also be configured to, based on the second cognitive state estimate 138, the second task list 127, and the one or more second user metrics 130, assign a second task 142 from the set of assignable tasks 134 to the second operator. In some examples, the first task 140 and the second task 142 can be different tasks from the set of assignable tasks 134. In the same or alternative examples, the task assignor 114 can determine whether to assign a task from the set of assignable tasks 134 to the first operator or the second operator.

In some examples, the task assignor 114 can also be configured to obtain (e.g., receive or retrieve) the set of assignable tasks 134, or a subset thereof, and, for each task included in the set, determine one or more task metrics for the task. The task metrics can include a task duration, a task skill level, one or more related tasks, or a combination thereof. In some examples, the task assignor 114 can generate task metric data. In the same or alternative examples, the task assignor 114 can obtain (receive or retrieve) task metric data from another source, such as the task database 118 via the task data 133. The task assignor 114 can be configured to select the first task 140 from the set of assignable tasks 134 based on the one or more task metrics for the first task 140.

In some examples, the task assignor 114 (or other appropriate component of the device 104) can also be configured to identify a cost function for each task of the set of assignable tasks 134. The cost function can include one or more cost metrics. The cost metrics can include, for example, a speed of completion, a time of completion, a skillset match, a quality level, a task accuracy, a cognitive state impact, or a combination thereof. The cost function can take into account one or more such cost metrics. In some examples in which the cost function considers a plurality of cost metrics, the cost function can indicate, for each cost metric, a weight value for each cost metric.

In some examples, the task assignor 114 can be configured to perform a comparative analysis based on the respective tasks lists for a plurality of operators 120 (e.g., the first task list 124 and the second task list 127). The task assignor 114 can be configured to assign the first task 140 to a particular operator based on a result of the comparative analysis. For example, after considering the cost metrics and cost functions associated with a particular task, as well as the cognitive state estimates and user metrics for each operator, the task assignor 114 can compare the list of tasks currently assigned to each operator and determine whether one operator is more suited than the other. To illustrate, a particular task can have associated cost data that indicates that there is a speed of completion cost metric and a time of completion metric, but that the speed of completion cost metric should be considered twice as heavily as the time of completion metric.

For each task, the task assignor 114 (or other appropriate component of the device 104) can be configured to determine a cost associated with the task based on the cost function identified for the task and based on the cognitive state estimate for the particular operator. The task assignor 114 can then select the first task 140 from the set of assignable tasks 134 based on one or more costs determined for the set of assignable tasks 134. For example, a particular task may have a time restriction indicating the particular task must be completed within a week. The task lists for a set of operators 120 (e.g., the first task list 124 and the second task list 127) may indicate that, while both operators are qualified and their respective cognitive state estimates do not currently indicate cognitive fatigue, a first operator has assigned tasks that will take longer than one week to complete, while a second operator does not.

In some examples, the device 104 can be configured to perform a Partially Observable Markov Decision Process (POMDP) as part of the task assignment. Other algorithms that account for the uncertainty of the input data can also be applied without departing from the scope of the subject disclosure. In some examples, the POMDP can also be applied in conjunction with the cost metrics described above. For example, the task assignor 114 can be configured to, to assign the first task 140, perform a POMDP based on the cognitive state estimate for a particular operator and the cost associated with the first task 140. If the output of the POMDP indicates that the particular operator is suffering from mental fatigue, the device 104 can be configured to assign a rest task to the particular operator, reassign one or more tasks from the respective task list to another operator, or a combination thereof.

The task assignor 114 can also be configured to generate data 128 and send the data 128 to the task assignment indicator generator 116. The task assignment indicator generator 116 can be configured to receive the data 128 from the task assignor 114 and generate output data 132 that can include dynamic, real-time task assignment information that can be passed on one or more operators 120, supervisors, tracking software, etc. For example, the output data 132 can include a first task assignment indicator 148 for communication to the interface device 106. The interface device 106 can include a human-machine interface 144, which can in some examples include an output generator 146. The interface device 106 can include an electronic device such as a computing device, portable computing device, wearable electronic device, etc.

The human-machine interface 144 can include an appropriate electronic means of allowing a human (e.g., one or more operators 120) to interface with the interface device 106, such as a keyboard, mouse, touchscreen, display screen, etc. The output generator 146 can be configured to receive data associated with the first task assignment indicator 148 and produce an output based on the first task assignment indicator 148 to indicate to a particular operator 120 to which the first task has been assigned. **In** this manner, the output data 132 can be displayed, via the human-machine interface 144 and the output generator 146, in various readable formats that allow for quick interpretation of the task assignment data.

In some examples, the device 104 can also be configured to communicate the output data 132 to the interface device 106. For example, the device 104 can be configured to communicate cognitive state estimate data associated with one or more operators 120 to the interface device 106 for consumption by the operator(s) 120, supervisors, other appropriate personnel, or a combination thereof.

In some examples, the interface device 106 can be configured to receive the first task assignment indicator 148 and generate, based on the first task assignment indicator 148, an output that indicates the identified first task 140 is assigned to the first operator, included in the first task list 124, or a combination thereof. In some examples, the interface device 106 can also be configured to indicate the first task 140 as a prioritized list that includes the first task 140, receive an input from the first operator that indicates a confirmation of the first task 140 included in the first task list 124, and send the input to the processor(s) 108 of the device 104.

The above example illustrates one manner in which the operator(s) 120 can provide feedback to the device 104 via the human-machine interface 144 of the interface device 106. As another example, at least one sensor of the sensor(s) 102 can be included in the interface device 106, the interface device 106 can include a heads-up display, or some combination thereof.

As a further example, the set of assignable tasks 134 can include tasks associated with one or more flight operation tasks for an aircraft. The device 104 can be configured to receive a completion indicator from the operator 120 via the human-machine interface 144 of the interface device 106 that indicates completion of the first task 140. The device 104 can also be configured to determine an aircraft state responsive to completion of the first task (e.g., that a particular course has been set).

In some examples, the cognitive state estimate data can include a description of an operator's 120 current cognitive state, a visualization of the cognitive state estimate, or a combination thereof. For example, the device 104 can include a data visualization generator configured to generate the output data 132 to include a visualization of the probabilistic outputs of the Multiple Model Cognitive State Estimator algorithm. The visualization can represent the GMM algorithm as a probability distribution curve or as confidence intervals around point estimates, providing a visualization of both the estimated cognitive states and the associated uncertainties. The data visualization generator can also be configured to generate the output data 132 to include composite displays that integrate multiple data streams from the data 128. For example, the data visualization generator can combine cognitive state estimates with gaze data (e.g., physiological data 122A) and task data 133 to provide a comprehensive view of the operator's current condition and awareness in relation to the assignable tasks.

In some examples, the data visualization generator can be configured to send the output data 132 to the memory 110. This allows for the archiving of the output data 132 for later analysis, training purposes, or other long-term studies on operator performance and cognitive patterns. By storing the output data 132, the system 100 enables more comprehensive retrospective analyses and continuous improvement of operator training and support systems.

During operation, the sensors 102 measure and collect physiological data 122 (e.g., physiological data) associated with the operator 120, including eye movements, heart rate, skin responses, audio, or a combination thereof. The task database 118 can also obtain task data from one or more sources. These data streams (e.g., physiological data 122 and task data 133) are then sent to the processor 108, such as a respective processor 108A-E. The task data 133 can also be sent to a task data processor. The processors 108 can process the physiological data 122, the task data 133, or a combination thereof, to clean and/or extract relative features from the physiological data 122, the task data 133, or a combination thereof.

The processed data 126 can then sent to the memory 110 for archiving, to the cognitive state estimator 112, or a combination thereof. The cognitive state estimator 112, which includes one or more algorithms for human cognitive state estimation, analyzes the processed data 126 (e.g., incoming data streams). The cognitive state estimator 112 generates estimates of the operator's 120 cognitive states, including mental workload, fatigue, attention allocation, mental processing resources, awareness of the operational system, or a combination thereof.

The cognitive state estimator 112 generates data associated with one or more of the operator's 120 cognitive states (including mental workload, fatigue, attention allocation, mental processing resources, awareness of the operational system, or a combination thereof) and provides the generated data to the task assignor 114. The task assignor 114 analyzes the tasks currently assigned to the operators 120, the set of assignable tasks 134, and the operator's 120 cognitive state estimates, to generate the data 128 which includes task assignment data. The data 128 is provided to the task assignment indicator generator 116. The task assignment indicator generator 116 takes the data 128 and transforms it into meaningful task assignment indicator(s). The task assignment indicator(s) can provide dynamic, real-time information regarding assignment of a particular task to a particular operator, generating output data 132.

The output data 132 is sent to the interface device 106, which presents the task assignment in a format that is interpretable by the operator(s) 120, supervisory personnel, tracking software, etc., or a combination thereof. The format may include a visual alert, audio alert, text, charts, etc., or a combination thereof, that allows for quick understanding of the task assignment.

The technical advantages of using the system 100 includes providing a comprehensive, real-time assessment of operator cognitive states and task awareness, which was previously difficult to obtain non-invasively in operational settings. This can significantly enhance efficiency and efficacy of certain operational environments in which an operator's current cognitive state can impact their ability to perform a particular task. For example, instead of assigning a new task to an operator once a previous task is complete, the system 100 allows for more appropriate task assignment by considering the individual operator's dynamically changing cognitive workload (e.g., mental fatigue) in a systematic, data-driven manner, which can thereby improve operation and/or efficiency of a task assignment system (e.g., an automated task assignment system).

Another technical advantage includes the system's 100 ability to process multiple data streams simultaneously and fuse them into meaningful insights. By combining physiological data with task data, the system 100 provides a more holistic view of the operator's performance and awareness than traditional monitoring methods.

Another technical advantage includes the real-time visualization capabilities that make complex data easily interpretable, enabling quick decision-making by supervisory crew or researchers. This is particularly valuable in identifying and mitigating instances of cognitive issues that might otherwise go unnoticed.

Another technical advantage includes the system's 100 data logging, replay capabilities, and reprocessing capabilities, which provide valuable tools for post-hoc analysis, training, and system improvement. This feature allows for detailed examination of operator performance and system behavior, which can inform future training protocols and system enhancements. The reprocessing capabilities allow raw data to be passed back through the system using new or updated models to generate higher quality output. This means that as algorithms and models are refined over time, historical data can be reanalyzed to yield new insights or improved accuracy, maximizing the value of collected data and enabling continuous improvement of the system's performance.

Another technical advantage includes that the GMM enables the device 104 to represent complex, multi-modal probability distributions that can capture the nuances of different cognitive states. By using multiple Gaussian components, the GMM can represent multiple hypotheses about the operator's state simultaneously, with the weights of these components reflecting the relative likelihood of each hypothesis. This approach is particularly useful in situations where the operator's cognitive state can be ambiguous or rapidly changing. The GMM also provides a way to incorporate uncertainty into the estimates, which is important for making robust decisions based on these cognitive state assessments.

FIG. 2 is an example 200 of an algorithm(s) estimator 210 that includes a Multi-Modal Cognitive State Estimation Framework. The algorithm(s) estimator 210 can include the cognitive state estimator 112 as described in FIG. 1.

The algorithm(s) estimator 210 can be configured to use one or more data 202 inputs. The data 202 can include data 202A, data 202B, and data 202C, each of which can represent various combinations of data types described in FIG. 1. These data types include the physiological data 122, the task data 133, the processed data 126, or any combination thereof. Each data (202A, 202B, 202C) input can contain different combinations of these data types, allowing for flexible and comprehensive analysis by the algorithm(s) estimator 210.

Each of the data 202 inputs is processed by a machine learning model 204, such as a respective machine learning model 204. The first model, machine learning model 204A, can employ Bayesian inference techniques. Bayesian inference is a statistical method that updates the probability of a hypothesis as more evidence becomes available. In this context, it could be used to estimate the likelihood of various operator states or conditions based on the data. For example, the machine learning model 204A can be configured to calculate the probability of operator fatigue given observed physiological signals, flight duration, and time of day. The machine learning model 204A using the Bayesian inference methods can provide a technical advantage in that it can handle uncertainty and incorporate prior knowledge about typical operator behavior or physiological responses.

Machine learning model 204B can be configured to utilize a neural network architecture. Neural networks are inspired by the human brain and consist of interconnected nodes organized in layers. The machine learning model 204B can be configured to identify complex patterns in the data 202. For example, the machine learning model 204B can be trained to recognize patterns in operator actions, eye movements, or physiological data that are indicative of certain cognitive states or levels of situational awareness.

Machine learning model 204C can be configured to use one or more regression models. The regression models can be configured to understand/identify the relationships between variables and make predictions. For example, the regression models used by the machine learning model 204C can be used to predict continuous variables like stress levels, reaction times, or performance metrics based on various input factors. To illustrate, the machine learning model 204C can be configured to estimate an operator's current level of mental workload based on factors associated with the task data 133. The machine learning model 204C can be configured to quantify the impact of different factors on operator performance and cognitive state.

The outputs from these machine learning models 204 can then be combined, via data fusion 206, and output as data 208. The data 208 can then be used by the task assignor 114, the task assignment indicator generator 116, or a combination thereof to generate the output data 132, including the first task assignment indicator 148, which can be communicated to the interface device 106 and displayed on the human-machine interface 144, as described in FIG. 1.

Using this integrated data 208, the algorithm(s) estimator 210 (e.g., Multi-Modal Cognitive State Estimation Framework) enables the device (e.g., the device 104 of FIG. 1) to determine the available attentional resources of the operator based on the mental workload and mental fatigue. This assessment can be configured to combine outputs from the machine learning models 204 to estimate the operator's current cognitive capacity. The algorithm(s) estimator 210 can be configured to then determine the attention allocation of the operator. This process takes into account the previously calculated available attentional resources and incorporates gaze patterns derived from the data 202. The algorithm(s) estimator 210 can be configured to assess the operator's task engagement by combining the attention allocation data with the task data.

This multi-machine learning model 204 approach allows the algorithm(s) estimator 210 to provide a comprehensive assessment of the operator's cognitive state and performance. By integrating diverse data sources (e.g., data 202A-C) and employing multi-machine learning models 204A-C, the algorithm(s) estimator 210 can be configured to provide insights that contribute to improved task assignment operations and/or determination, enhanced safety and efficiency in aviation operations, or a combination thereof.

In some examples, the machine learning models 204A-C can be configured to use various types of algorithms. For example, the machine learning models 204A-C could employ decision trees, random forests, support vector machines, gradient boosting machines, or deep learning architectures such as convolutional neural networks (CNNs) or recurrent neural networks (RNNs). The choice of model for each input can be based on the characteristics of the data and the particular aspect of an operator cognitive state being estimated. In some examples, the machine learning models 204A-C can be of the same type, such as all being neural networks or all being another specific type of machine learning model (e.g., regression models such as Gaussian processes). Different models, even of the same type, can learn differently and provide varied outputs due to differences in their architecture, training data, or hyperparameters. This flexibility enables the system 200 to be optimized for different scenarios or types of data inputs.

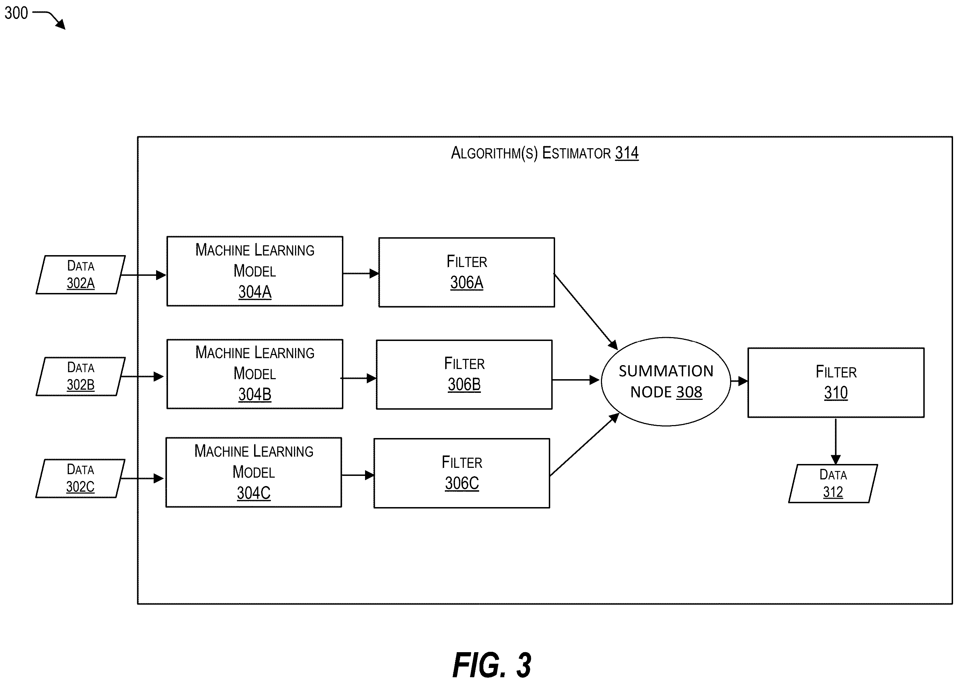

FIG. 3 is a diagram 300 that illustrates a particular example of an algorithm(s) estimator 314 that includes a Gaussian Mixture Model (GMM) algorithm. The algorithm(s) estimator 314 can be configured to process multiple streams of data to estimate various cognitive states of the operator. The algorithm(s) estimator 314 can include the cognitive state estimator 112 as described in FIG. **1****,** the algorithm(s) estimator 210 as described in FIG. 2, or a combination thereof.

The algorithm(s) estimator 314 can be configured to use one or more data 302 inputs. The data 302 can include data 302A, data 302B, and data 302C, each of which can represent various combinations of data types described in FIG. 1. These data types include the physiological data 122, the task data 133, the processed data 126, or any combination thereof. Each data (302A, 302B, 302C) input can include different combinations of these data types, thereby allowing for flexible and comprehensive analysis by the algorithm(s) estimator 314.

Each of the data 302 is processed by a separate machine learning model 304. For example, the data 302A is processed by machine learning model 304A, the data 302B is processed by machine learning model 304B, and the data 302C is processed by the machine learning model 304C. Each of these machine learning models 304 can be configured to implement an Expectation Maximization (EM) algorithm. In some examples, the machine learning models 304 can be configured to use neural networks, support vector machines, random forests, gradient boosting machines, hidden Markov models, or a combination thereof, based on the specific characteristics of the data 302 and the desired outputs.

In some examples, the machine learning models 304 can be configured to employ the EM algorithm for training. The EM algorithm can be an iterative method that enables the machine learning models 304 to learn the relationship between the data 302 (e.g., physiological signals, eye movements) and the corresponding cognitive states (e.g., workload, fatigue, attention). In some examples, the EM algorithm can estimate the mean and covariance of Gaussian distributions that represent the likelihood of a particular cognitive state given the data 302. The EM algorithm can iteratively refine these estimates by computing the expected value of the log-likelihood function and maximizing it with respect to the mean and covariance. The EM algorithm can continue this process until the model(s) 304 converge to a maximum likelihood estimate of the mean and covariance, thereby enhancing the accuracy and reliability of the cognitive state estimation. The outputs of these machine learning models 304 can then be sent to filters 306A-C.

In some examples, the machine learning models 304 can be configured to determine patterns and relationships within the data 302. Each machine learning model 304 can be configured to identify key features and map these features to cognitive state estimates. For example, the machine learning model 304A can be configured to estimate mental workload based on heart rate variability and eye movement patterns, while another machine learning model 304B can be configured to estimate fatigue based on blink rate and vocal characteristics.

The outputs of the machine learning models 304 can then be provided to filters 306A-C. These filters 306 can be configured to include Kalman filters. In some examples, the filters 306 can be configured to include particle filters, unscented Kalman filters, extended Kalman filters, H-infinity filters, or a combination thereof. Each Kalman filter can be configured to, based on physiological data (e.g., the physiological data 122 of FIG. 1), generate an output that indicates a candidate cognitive state of the operator (e.g., the operator(s) 120 of FIG. 1).

Each of the filters 306 can be configured to refine and smooth the estimates produced by the machine learning models 304. The filters 306 can be configured to take into account the temporal aspects of the data, thereby reducing noise and providing more stable estimates over time. For example, a filter 306 can be configured to smooth out rapid fluctuations in estimated workload that are likely due to measurement noise rather than actual changes in cognitive state.

The outputs of these individual filters 306 can then be combined at a summation node 308. The summation node 308 can be configured to aggregate the estimates from the different data streams, potentially applying weights to prioritize certain estimates over others based on their reliability or relevance.

The aggregated estimates produced by the summation node 308 can be processed by a filter 310. The filter 310 can be configured to include a Gaussian Mixture Model (GMM) algorithm. The filter 310 can be configured to determine the uncertainty in the estimates and potentially represent multiple hypotheses about the operator's cognitive state. The output of the filter 310 can be represented as data 312, which can include or indicate the final estimates of the operator's cognitive states.

During operation, the algorithm(s) estimator 314 can include a GMM filter algorithm that can be configured to determine the available attentional resources of the operator based on the mental workload and mental fatigue estimates. By combining estimates of workload and fatigue from the various data 302, the algorithm(s) estimator 314 can infer an attentional resource capacity/availability of the operator at a given time. For example, the algorithm(s) estimator 314 can be configured to combine, based on the GMM, the outputs of multiple Kalman filters to generate a combined output, and to output, based on the combined output of the multiple Kalman filters, the cognitive state estimate of the operator(s).

In some examples, to generate the combined output of the multiple Kalman filters, the algorithm(s) estimator 314 can be configured to, for the output of each Kalman filter, apply a weight value to the output of the Kalman filter. In some examples, the weight value applied is based on the physiological data (e.g., the physiological data 122 of FIG. 1). For example, the algorithm(s) estimator 314 can apply a higher valued weight (e.g., a heavier weight) to physiological data 122A from the eye tracker sensor 102A of FIG. 1 than to the physiological data 122E from the microphone sensor 102E when generating a cognitive state estimate for task engagement of a particular operator 120.

The algorithm(s) estimator 314 can be configured to determine the attention allocation of the operator based on the available attentional resources and gaze patterns derived from the physiological data (e.g., data 302). By analyzing where the operator is looking (from the data 302) in the context of their available attentional resources, the algorithm(s) estimator 314 can estimate how the operator is distributing their attention across different task items. The algorithm(s) estimator 314 can be configured to determine task engagement of the operator based on the attention allocation and the task data (e.g., data 302). By combining information (e.g., at summation node 308) about where the operator is allocating their attention with data (e.g., the data 302) about the current task data (such as the first task list 124, second task list 127, first user metrics 129, second user metrics 130 of FIG. 1, etc.), the algorithm(s) estimator 314 can estimate the operator's current task engagement.

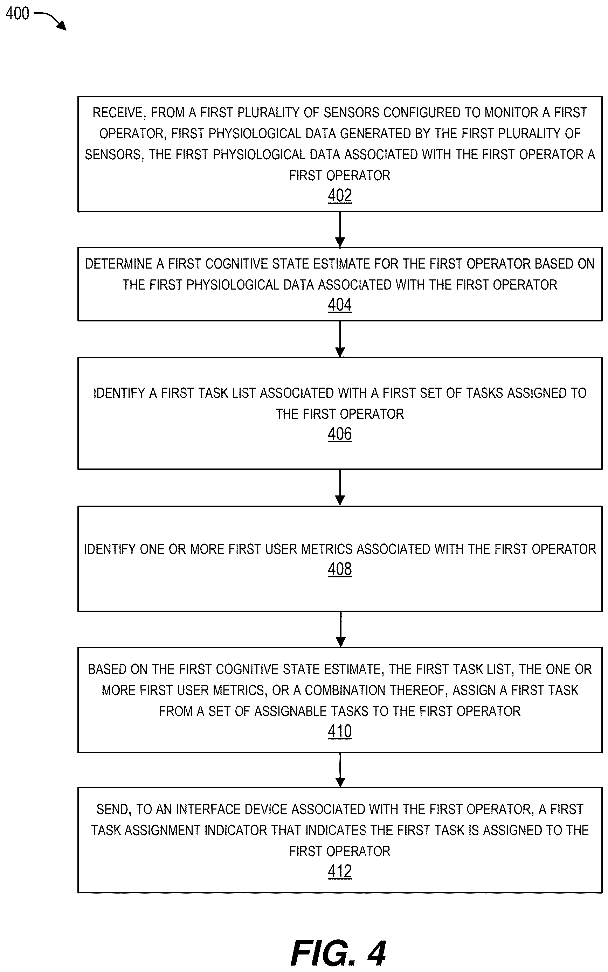

FIG. 4 is a flow chart of a method 400 of use of an automatic task assignment system. The method 400 may be performed by the system 100, the device 104, the processor 108, or a combination thereof.

The method 400 includes, at block 402, receiving, from a first plurality of sensors configured to monitor a first operator, first physiological data generated by the first plurality of sensors. The first physiological data may be associated with the first operator. For example, the system 100 of FIG. 1 can be configured to receive, from the sensors 102 configured to monitor a first operator of the operators 120, the physiological data 122 generated by the sensors 102.

The method 400 also includes, at block 404, determining a first cognitive state estimate for the first operator based on the first physiological data associated with the first operator. For example, the system 100 of FIG. 1 can be configured to determine (e.g., by or using the cognitive state estimator 112) the first cognitive state estimate 136 based on the physiological data 122 associated with the first operator.

The method 400 also includes, at block 406, identifying a first task list associated with a first set of tasks assigned to the first operator. For example, the system 100 of FIG. 1 can be configured to identify (e.g., by or using the task assignor 114) the first task list 124 from the task data 133, where the first task list 124 is associated with a first set of tasks assigned to the first operator.

The method 400 also includes, at block 408, identifying one or more first user metrics associated with the first operator. For example, the system 100 of FIG. 1 can be configured to identify (e.g., by or using the task assignor 114) the first user metrics 129 from the task data 133, where the first user metrics 129 are associated with the first operator.

The method 400 also includes, at block 410, based on the first cognitive state estimate, the first task list, the one or more first user metrics, or a combination thereof, assigning a first task from a set of assignable tasks to the first operator. For example, the system 100 of FIG. 1 can be configured to assign (e.g., by or using the task assignor 114), based on the first task list 124, the first user metrics 129, the first cognitive state estimate 136, or a combination thereof, the first task 140 from the set of assignable tasks 134 to the first operator.

The method 400 also includes, at block 412, sending, to an interface device associated with the first operator, a first task assignment indicator that indicates the first task is assigned to the first operator. For example, the system 100 of FIG. 1 can be configured to send (e.g., by or using the device 104), to the interface device 106 associated with the first operator, the first task assignment indicator 148 that indicates the first task 140 is assigned to the first operator.

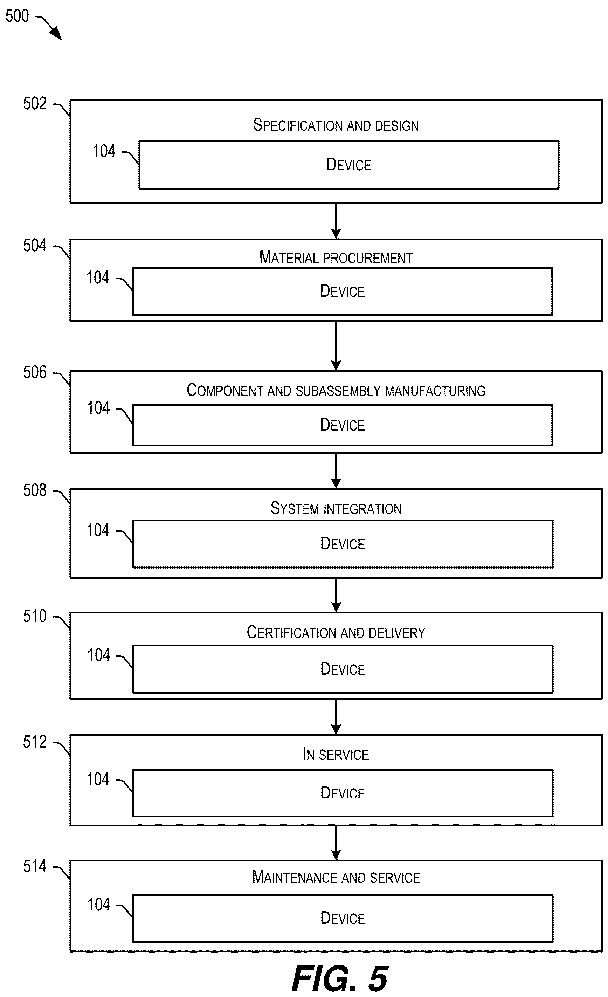

FIG. 5 is a flowchart illustrating an example 500 of a life cycle of an aircraft that includes the automatic task assignment system 100 of FIG. 1. During pre-production, the exemplary method 500 includes, at block 502, specification and design of the aircraft. During specification and design of the aircraft, the method 500 may include specification and design of the device 104 and locations where the device 104 are to be placed. At block 504, the method 500 includes material procurement, which may include procuring materials for the device 104 or procuring a pre-assembled device 104.

During production, the method 500 includes, at block 506, component and subassembly manufacturing and, at block 508, system integration of the aircraft. For example, the method 500 may include component and subassembly manufacturing of the device 104, system integration of the device 104 with the aircraft, or both. At block 510, the method 500 includes certification and delivery of the aircraft and, at block 512, placing the aircraft in service. Certification and delivery may include certification of the device 104 to place the device 104 in service. While in service by a customer, the aircraft may be scheduled for routine maintenance and service (which may also include modification, reconfiguration, refurbishment, and so on). At block 514, the method 500 includes performing maintenance and service on the aircraft, which may include performing maintenance and service on the device 104. For example, the maintenance and service can include updating one or more algorithms used by the estimation algorithm, replacing one or more processors 108, or a combination thereof.

Each of the processes of the method 500 may be performed or carried out by a system integrator, a third party, and/or an operator (e.g., a customer). For the purposes of this description, a system integrator may include without limitation any number of aircraft manufacturers and major-system subcontractors; a third party may include without limitation any number of venders, subcontractors, and suppliers; and an operator may be an airline, leasing company, military entity, service organization, and so on.

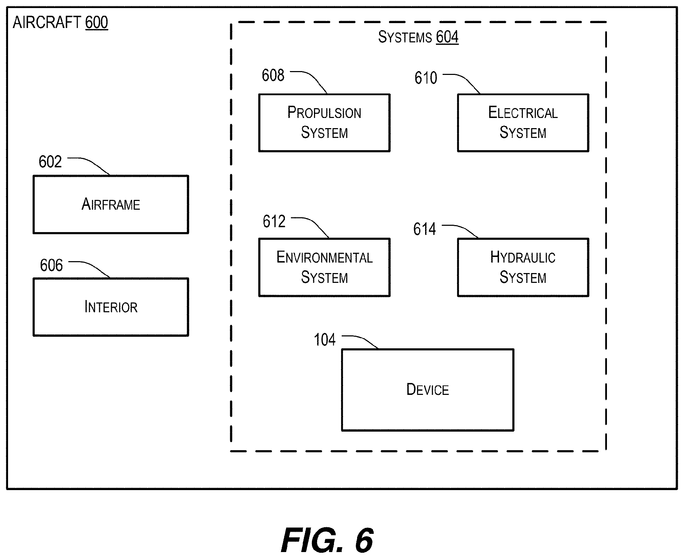

Examples of the disclosure can be described in the context of an example of an aircraft 600 as shown in FIG. 6. In the example of FIG. 6, the aircraft 600 includes an airframe 602 with a plurality of systems 604 and an interior 606. Examples of the plurality of systems 604 include one or more of a propulsion system 608, an electrical system 610, an environmental system 612, a hydraulic system 614, and the device 104 (and/or the system 100 or a portion thereof). Any number of other systems may be included. In the example of FIG. 6, the aircraft 600 includes the device 104 in accordance with one or more examples of the disclosure as described in FIGS. 1-5. Portions of the device 104 are included in the airframe 602 and the interior 606. Also, the device 104 utilizes portions of the electrical system 610. For example, the device 104 may be powered by the electrical system 610.

FIG. 7 is a block diagram of a computing environment 700 including a computing device 710 configured to support examples of computer-implemented methods and computer-executable program instructions (or code) according to the subject disclosure. For example, the computing device 710, or portions thereof, can be configured to execute instructions to initiate, perform, or control one or more operations described with reference to FIGS. 1-6.

The computing device 710 includes one or more processors 720. In some examples, the processor(s) 720 includes the processor(s) 108, as described in FIGS. 1-6. The processor(s) 720 are configured to communicate with system memory 730, one or more storage devices 740, one or more input/output interfaces 750, one or more communications interfaces 760, or any combination thereof. The system memory 730 includes volatile memory devices (e.g., random access memory (RAM) devices), nonvolatile memory devices (e.g., read-only memory (ROM) devices, programmable read-only memory, and flash memory), or both. The system memory 730 stores an operating system 732, which may include a basic input/output system for booting the computing device 710 as well as a full operating system to enable the computing device 710 to interact with users, other programs, and other devices. The system memory 730 stores system (program) data 736, such as the physiological data 122, the task data 133, the processed data 126, the data 128, the output data 132 of FIG. 1, the data 202 of FIG. 2, the data 302 of FIG. 3, or a combination thereof.

The system memory 730 includes one or more operating systems 732 and/or one or more applications 734 (e.g., sets of instructions) executable by the processor(s) 720. As an example, the one or more applications 734 include instructions executable by the processor(s) 720 to initiate, control, or perform one or more operations described with reference to FIGS. 1-6, such as determining mental workload and mental fatigue of an operator based on physiological data, determining available attentional resources of the operator based on the mental workload and the mental fatigue, determining attention allocation of the operator based on the available attentional resources and gaze patterns derived from the physiological data, determining task engagement of the operator based on the attention allocation and task data, and generating a task assignment indicator. The applications 734 can include the task assignment indicator generator 116, the cognitive state estimator 112, the task assignor 114 of FIG. 1, the algorithm(s) estimator 210 of FIG. 2, the algorithm(s) estimator 314 of FIG. 3, or a combination thereof.

In some examples, the system memory 730 includes a non-transitory, computer-readable medium storing the instructions that, when executed by the processor(s) 720, cause the processor(s) 720 to initiate, perform, or control operations to aid in design of an object. The operations include determining mental workload and mental fatigue of an operator based on physiological data, determining available attentional resources of the operator based on the mental workload and the mental fatigue, determining attention allocation of the operator based on the available attentional resources and gaze patterns derived from the physiological data, determining task engagement of the operator based on the attention allocation and task data, and generating a task assignment indicator.

The one or more storage devices 740 include nonvolatile storage devices, such as magnetic disks, optical disks, or flash memory devices. In some particular examples, the storage devices 740 include both removable and non-removable memory devices. The storage devices 740 are configured to store an operating system, images of operating systems, applications (e.g., one or more of the applications 734), and program data (e.g., the program data 736). In some examples, the system memory 730, the storage devices 740, or both, include tangible computer-readable media. In some examples, the system memory 730, the storage device 1740, or a combination thereof, include or correspond to the task database 118 of FIG. 1. In some examples, one or more of the storage devices 740 are external to the computing device 710.

The one or more input/output interfaces 750 enable the computing device 710 to communicate with one or more input/output devices 770 to facilitate user interaction. For example, the input/output interface 750 is adapted to receive input from a user, to receive input from another computing device, or a combination thereof. In some examples, the input/output interface 750 conforms to one or more standard interface protocols, including serial interfaces (e.g., universal serial bus (USB) interfaces or Institute of Electrical and Electronics Engineers (IEEE) interface standards), parallel interfaces, display adapters, audio adapters, or custom interfaces ("IEEE" is a registered trademark of The Institute of Electrical and Electronics Engineers, Inc. of Piscataway, New Jersey). In some examples, the input/output device 770 includes one or more user interface devices and displays, including some combination of buttons, keyboards, pointing devices, displays, speakers, microphones, touch screens, or other device.

The processor(s) 720 are configured to communicate with devices or controllers 780 via the one or more communications interfaces 760. For example, the one or more communications interfaces 760 can include a network interface. In other examples, the one or more devices or controllers 780 includes the sensor(s) 102.

In some examples, a non-transitory, computer-readable medium stores instructions that, when executed by one or more processors, cause the one or more processors to initiate, perform, or control operations to perform part or all of the functionality described above. For example, the instructions may be executable to implement one or more of the operations or methods of FIGS. 1-6. In some examples, part, or all of one or more of the operations or methods of FIGS. 1-6 may be implemented by one or more processors (e.g., one or more central processing units (CPUs), one or more graphics processing units (GPUs), one or more digital signal processors (DSPs)) executing instructions, by dedicated hardware circuitry, or any combination thereof.

Particular examples of the disclosure are described below.

According to Example 1, a system for automatically assigning tasks includes a first plurality of sensors configured to monitor a first operator and generate first physiological data associated with the first operator. The system also includes one or more processors configured to determine a first cognitive state estimate for the first operator based on the first physiological data associated with the first operator. The one or more processors are also configured to identify a first task list associated with a first set of tasks assigned to the first operator. The one or more processors are further configured to identify one or more first user metrics associated with the first operator. The one or more processors are also configured to, based on the first cognitive state estimate, the first task list, and the one or more first user metrics, assign a first task from a set of assignable tasks to the first operator. The one or more processors are also configured to send, to an interface device associated with the first operator, a first task assignment indicator that indicates the first task is assigned to the first operator.

Example 2 includes the system of Example 1, where the first plurality of sensors includes an eye tracker, a thermal camera, an electrodermal response sensor, a microphone, or a heart rate monitor, or a combination thereof. Example 2 also includes the system of Example 1, where the first cognitive state estimate indicates a cognitive workload of the first operator, a fatigue of the first operator, a task engagement level, or a combination thereof. Example 2 also includes the system of Example 1, where the first task is selected from the set of assignable tasks based on the first cognitive state estimate, the first task list, and the one or more first user metrics.

Example 3 includes the system of Example 1 or Example 2, where, to determine the first cognitive state estimate, the one or more processors are further configured to, for each Kalman filter of multiple Kalman filters and based on the first physiological data, generate an output of the Kalman filter that indicates a candidate cognitive state of the first operator. The one or more processors are also configured to combine, based on a Gaussian Mixture Model (GMM), the outputs of the multiple Kalman filters to generate a combined output; and output, based on the combined output of the multiple Kalman filters, the first cognitive state estimate for the first operator.

Example 4 includes the system of Example 3, where to generate the combined output of the multiple Kalman filters, the one or more processors are further configured to, for the output of each Kalman filter of the multiple Kalman filters, apply a weight value to the output of the Kalman filter, and the weight value applied to each respective Kalman filter is based on the first physiological data.

Example 5 includes the system of any of Examples 1 to 4, where the first cognitive state estimate indicates, in association with the first operator, an attention allocation, available mental processing resources, an awareness of an operational system, a fatigue level, or a combination thereof. Example 5 also includes the system of any of Examples 1 to 4, where the one or more first user metrics include a skill level, a task completion speed, a cognitive state range, a workload range, or a combination thereof. Example 5 also includes the system of any of Examples 1 to 4, where the first task list indicates a working task, a waiting task, or a combination thereof.

Example 6 includes the system of any of Examples 1 to 5, and further includes the interface device associated with the first operator, where the interface device includes a human-machine-interface device and is configured to receive the first task assignment indicator. The interface device is also configured to generate, based on the first task assignment indicator, an output that indicates the identified first task is assigned to the first operator, included in the first task list, or a combination thereof.

Example 7 includes the system of Example 6, where the interface device is further configured to indicate the first task as a prioritized list that includes the first task. The interface device is also configured to receive an input from the first operator that indicates a confirmation of the first task included in the first task list. The interface device is also configured to send the input to the one or more processors.

Example 8 includes the system of Example 6 or Example 7, where at least one sensor of the first plurality of sensors is included in the interface device, the interface device includes a heads up display, or a combination thereof.

Example 9 includes the system of any of Examples 1 to 8, where the one or more processors are further configured to receive the set of assignable tasks. The one or more processors are also configured to, for each task included in the set of assignable tasks, determine one or more task metrics for the task, the one or more respective task metrics include a task duration, a task skill level, one or more related tasks, or a combination thereof. The first task is selected from the set of assignable tasks further based on the one or more task metrics for the first task.

Example 10 includes the system of any of Examples 1 to 9, where, to assign the first task, the one or more processors are further configured to perform a Partially Observable Markov Decision Process (POMDP).

Example 11 includes the system of any of Examples 1 to 10, where the one or more processors are further configured to, for each task of the set of assignable tasks, identify a cost function for the task. The cost function includes one or more cost metrics. The one or more processors are also configured to, for each task of the set of assignable tasks, determine a cost associated with the task based on the cost function identified for the task and based on the first cognitive state estimate for the first operator. The one or more processors are also configured to, for each task of the set of assignable tasks, select the first task from the set of assignable tasks based on one or more costs determined for the set of assignable tasks.

Example 12 includes the system of Example 11, where, to assign the first task, the one or more processors are further configured to perform a POMDP based on the first cognitive state estimate and the cost associated with the task. The one or more processors are further configured to, based on an output of the POMDP, assign a rest task to the first operator, reassign one or more tasks from the first task list to another operator, or a combination thereof.

Example 13 includes the system of Example 11 or Example 12, where the one or more cost metrics include a speed of completion, a time of completion, a skillset match, a quality level, a task accuracy, a cognitive state impact, or a combination thereof. The cost function indicates, for each cost metric of the one or more cost metrics, a weight value of the cost metric.

Example 14 includes the system of any of Examples 1 to 13, and further includes a second plurality of sensors configured to monitor a second operator; and generate second physiological data associated with the second operator. The one or more processors are further configured to determine a second cognitive state estimate for the second operator based on the second physiological data associated with the second operator; identify a second task list associated with a second set of tasks assigned to the second operator; and identify one or more second user metrics associated with the first operator, the first task is assigned to the first operator further based on the second cognitive state estimate, the second task list, the one or more second user metrics, or a combination thereof.

Example 15 includes the system of Example 14, where the one or more processors are configured to perform a comparative analysis based on the first task list and the second task list; and the first task is assigned to the first operator further based on a result of the comparative analysis.

Example 16 includes the system of any of Examples 1 to 15, where the set of assignable tasks are associated with one or more flight operation tasks for an aircraft; and the one or more processors are further configured to receive and a completion indicator that indicates completion of the first task; and determine an aircraft state responsive to completion of the first task.

According to Example 17, a method includes receiving, from a first plurality of sensors configured to monitor a first operator, first physiological data generated by the first plurality of sensors, the first physiological data associated with the first operator. The method also includes determining a first cognitive state estimate for the first operator based on the first physiological data associated with the first operator. The method also includes identifying a first task list associated with a first set of tasks assigned to the first operator. The method further includes identifying one or more first user metrics associated with the first operator. The method also includes, based on the first cognitive state estimate, the first task list, the one or more first user metrics, or a combination thereof, assigning a first task from a set of assignable tasks to the first operator. The method also includes sending, to an interface device associated with the first operator, a first task assignment indicator that indicates the first task is assigned to the first operator.

Example 18 includes the method of Example 17, and further includes displaying, at the interface device, the first task as a prioritized list that includes the first task; and receiving an input from the first operator that indicates a confirmation of the first task included in the first task list.

Example 19 includes the method of Example 17 or Example 18, where the first cognitive state estimate indicates, in association with the first operator, an attention allocation, available mental processing resources, an awareness of an operational system, a fatigue level, or a combination thereof. The one or more first user metrics include a skill level, a task completion speed, a cognitive state range, a workload range, or a combination thereof. The first task list indicates a working task, a waiting task, or a combination thereof.

According to Example 20, a non-transitory computer-readable medium storing instructions that, when executed by one or more processors, cause the one or more processors to receive, from a first plurality of sensors configured to monitor a first operator, first physiological data generated by the first plurality of sensors, the first physiological data associated with the first operator. The instructions, when executed by the one or more processors, also cause the one or more processors to determine a first cognitive state estimate for the first operator based on the first physiological data associated with the first operator. The instructions, when executed by the one or more processors, further cause the one or more processors to identify a first task list associated with a first set of tasks assigned to the first operator. The instructions, when executed by the one or more processors, cause the one or more processors to identify one or more first user metrics associated with the first operator. The instructions, when executed by the one or more processors, also cause the one or more processors to, based on the first cognitive state estimate, the first task list, the one or more first user metrics, or a combination thereof, assign a first task from a set of assignable tasks to the first operator. The instructions, when executed by the one or more processors, further cause the one or more processors to send, to an interface device associated with the first operator, a first task assignment indicator that indicates the first task is assigned to the first operator.

The illustrations of the examples described herein are intended to provide a general understanding of the structure of the various examples. The illustrations are not intended to serve as a complete description of all of the elements and features of apparatus and systems that utilize the structures or methods described herein. Many other examples may be apparent to those of skill in the art upon reviewing the disclosure. Other examples may be utilized and derived from the disclosure, such that structural and logical substitutions and changes may be made without departing from the scope of the appended claims. For example, method operations may be performed in a different order than shown in the figures or one or more method operations may be omitted. Accordingly, the disclosure and the figures are to be regarded as illustrative rather than restrictive.

Moreover, although specific examples have been illustrated and described herein, it should be appreciated that any subsequent arrangement designed to achieve the same or similar results may be substituted for the specific examples shown. This disclosure is intended to cover any and all subsequent adaptations or variations of various examples. Combinations of the above examples, and other examples not specifically described herein, will be apparent to those of skill in the art upon reviewing the description.

The Abstract of the Disclosure is submitted with the understanding that it will not be used to interpret or limit the scope or meaning of the claims. **In** addition, in the foregoing Detailed Description, various features may be grouped together or described in a single example for the purpose of streamlining the disclosure. Examples described above illustrate but do not limit the disclosure. **It** should also be understood that numerous modifications and variations are possible in accordance with the principles of the present disclosure. As the following claims reflect, the claimed subject matter may be directed to less than all of the features of any of the disclosed examples. Accordingly, the scope of the disclosure is defined by the following claims.

## Claims

1. A system (100) for automatically assigning tasks, comprising:
a first plurality of sensors (102) configured to:
monitor a first operator (120); and
generate first physiological data (122) associated with the first operator; and
one or more processors (108) configured to:
determine a first cognitive state estimate (136) for the first operator based on the first physiological data associated with the first operator;
identify a first task list (124) associated with a first set of tasks assigned to the first operator;
identify one or more first user metrics (129) associated with the first operator;
based on the first cognitive state estimate, the first task list, and the one or more first user metrics, assign a first task (140) from a set of assignable tasks (134) to the first operator; and
send, to an interface device (106) associated with the first operator, a first task assignment indicator (148) that indicates the first task is assigned to the first operator.

2. The system of claim **1,** wherein:
the first plurality of sensors includes an eye tracker (102A), a thermal camera (102B), an electrodermal response sensor (102D), a microphone (102E), or a heart rate monitor (102C), or a combination thereof; and
the first cognitive state estimate indicates a cognitive workload of the first operator, a fatigue of the first operator, a task engagement level, or a combination thereof; and
the first task is selected from the set of assignable tasks based on the first cognitive state estimate, the first task list, and the one or more first user metrics.

3. The system of claim 1 or 2, wherein, to determine the first cognitive state estimate, the one or more processors are further configured to:
for each Kalman filter of multiple Kalman filters and based on the first physiological data, generate an output of the Kalman filter that indicates a candidate cognitive state of the first operator;
combine, based on a Gaussian Mixture Model (GMM), the outputs of the multiple Kalman filters to generate a combined output; and
output, based on the combined output of the multiple Kalman filters, the first cognitive state estimate for the first operator; and, optionally, wherein:
to generate the combined output of the multiple Kalman filters, the one or more processors are further configured to
for the output of each Kalman filter of the multiple Kalman filters, apply a weight value to the output of the Kalman filter, and
the weight value applied to each respective Kalman filter is based on the first physiological data.

4. The system of any preceding claim, wherein:
the first cognitive state estimate indicates, in association with the first operator, an attention allocation, available mental processing resources, an awareness of an operational system, a fatigue level, or a combination thereof;
the one or more first user metrics include a skill level, a task completion speed, a cognitive state range, a workload range, or a combination thereof; and
the first task list indicates a working task, a waiting task, or a combination thereof.

5. The system of any preceding claim, further comprising:
the interface device associated with the first operator,
wherein the interface device includes a human-machine-interface device (144) and is configured to:
receive the first task assignment indicator; and
generate, based on the first task assignment indicator, an output that indicates the identified first task is: assigned to the first operator; and/or included in the first task list; and, optionally, wherein the interface device is further configured to:
indicate the first task as a prioritized list that includes the first task;
receive an input from the first operator that indicates a confirmation of the first task included in the first task list; and
send the input to the one or more processors.

6. The system of claim 5, wherein:
at least one sensor of the first plurality of sensors is included in the interface device;
the interface device includes a heads up display; or
a combination thereof.

7. The system of any preceding claim, wherein:
the one or more processors are further configured to:
receive the set of assignable tasks;
for each task included in the set of assignable tasks, determine one or more task metrics for the task, the one or more respective task metrics include a task duration, a task skill level, one or more related tasks, or a combination thereof; and
the first task is selected from the set of assignable tasks further based on the one or more task metrics for the first task.

8. The system of any preceding claim, wherein, to assign the first task, the one or more processors are further configured to perform a Partially Observable Markov Decision Process (POMDP).

9. The system of any preceding claim, wherein the one or more processors are further configured to, for each task of the set of assignable tasks:
identify a cost function for the task, the cost function includes one or more cost metrics;
determine a cost associated with the task based on the cost function identified for the task and based on the first cognitive state estimate for the first operator; and
select the first task from the set of assignable tasks based on one or more costs determined for the set of assignable tasks; and, optionally,
wherein, to assign the first task, the one or more processors are further configured to perform a POMDP based on the first cognitive state estimate and the cost associated with the task, and the one or more processors are further configured to, based on an output of the POMDP: assign a rest task to the first operator; and/or reassign one or more tasks from the first task list to another operator.

10. The system of claim 9, wherein:
the one or more cost metrics include a speed of completion, a time of completion, a skillset match, a quality level, a task accuracy, a cognitive state impact, or a combination thereof; and/or
the cost function indicates, for each cost metric of the one or more cost metrics, a weight value of the cost metric.

11. The system of any preceding claim, further comprising:
a second plurality of sensors configured to:
monitor a second operator; and
generate second physiological data associated with the second operator, and
wherein:
the one or more processors are further configured to:
determine a second cognitive state estimate for the second operator based on the second physiological data associated with the second operator;
identify a second task list associated with a second set of tasks assigned to the second operator; and
identify one or more second user metrics associated with the first operator,
the first task is assigned to the first operator further based on the second cognitive state estimate, the second task list, the one or more second user metrics, or a combination thereof; and, optionally,
wherein: the one or more processors are configured to perform a comparative analysis based on the first task list and the second task list; and the first task is assigned to the first operator further based on a result of the comparative analysis.

12. The system of any preceding claim, wherein:
the set of assignable tasks are associated with one or more flight operation tasks for an aircraft; and
the one or more processors are further configured to:
receive and a completion indicator that indicates completion of the first task; and
determine an aircraft state responsive to completion of the first task.

13. A method (400) comprising:
receiving (402), from a first plurality of sensors (102) configured to monitor a first operator (120), first physiological data (122) generated by the first plurality of sensors, the first physiological data associated with the first operator; and
determining (404) a first cognitive state estimate (136) for the first operator based on the first physiological data associated with the first operator;
identifying (406) a first task list (124) associated with a first set of tasks assigned to the first operator;
identifying (408) one or more first user metrics (129) associated with the first operator;
based on the first cognitive state estimate, the first task list, the one or more first user metrics, or a combination thereof, assigning (410) a first task (140) from a set of assignable tasks (134) to the first operator; and
sending (412), to an interface device (106) associated with the first operator, a first task assignment indicator (148) that indicates the first task is assigned to the first operator; and, optionally,
displaying, at the interface device, the first task as a prioritized list that includes the first task; and
receiving an input from the first operator that indicates a confirmation of the first task included in the first task list.

14. The method of claim 13, wherein:
the first cognitive state estimate indicates, in association with the first operator, an attention allocation, available mental processing resources, an awareness of an operational system, a fatigue level, or a combination thereof;
the one or more first user metrics include a skill level, a task completion speed, a cognitive state range, a workload range, or a combination thereof; and
the first task list indicates a working task, a waiting task, or a combination thereof.

15. A computer program comprising computer program instructions that, when executed by one or more computer processors, cause the one or more computer processors to perform the method of any preceding claim, or a non-transitory computer-readable medium (110, 730) having stored thereon such a computer program.
